# EUROPEAN PATENT APPLICATION

(11) **EP 3 483 287 A1**
(43) Date of publication of application: **15.05.2019**
(21) Application number: 18205948.5
(22) Date of filing: 28.01.2015
(51) Int. Cl.: C12Q 1/686

(54) **INTERNAL AMPLIFICATION CONTROL**

(30) Priority: 28.01.2014 EP 14152875
(62) Divisional of application: 15701773.2
(71) Applicant: QIAGEN GmbH, 40724 Hilden (DE)
(72) Inventor: Strauss, Sascha, 42697 Solingen (DE); Müller, Daniel, 40724 Hilden (DE); Siegmund, Vanessa, 40227 Düsseldorf (DE)
(74) Representative: CH Kilger Anwaltspartnerschaft mbB

(57) **Abstract**

Molecular biology techniques are widely used in genotyping applications and other areas such as biological research, forensic and diagnostic applications, including human identification and paternity testing and for diagnosis of infectious diseases or chimera analysis after allogeneic bone marrow transplantation as well the detection of genetic diseases and cancer. The most commonly used technique is the polymerase chain reaction (PCR) that allows the researchers to amplify the desired DNA requiring only tiny amounts of sample. Such amplification reactions are technically challenging and are often hampered by several practical issues such as the presence of PCR inhibitors, sample degradation and low quantities of said sample.

The invention addresses these issues by means of an internal amplification control consisting of a set of primers and an artificial template. The primers define two fragments of different sizes. The examples show that this system allows the researcher to distinguish between the presence of inhibitors and sample degradation.

SEQUENCE LISTING

<110>  Qiagen GmbH

<120>  Internal amplification control

<130>  B051-0148EP3

<150>  EP 14 15 2875.2
<151>  2014-01-28

<160>  34

<170>  PatentIn version 3.5

<210>  1
<211>  24
<212>  DNA
<213>  Artificial Sequence

<220>
<223>  Primer

<400>  1
tactccaacc acataatagt cctc                                              24


<210>  2
<211>  24
<212>  DNA
<213>  Artificial sequence

<220>
<223>  Primer

<400>  2
tctactccaa ccacataata gtcc                                              24


<210>  3
<211>  24
<212>  DNA
<213>  Artificial sequence

<220>
<223>  Primer

<400>  3
tcgctctact ccaaccacat aata                                              24


<210>  4
<211>  21
<212>  DNA
<213>  Artificial sequence

<220>
<223>  Primer

<400>  4
cagccgcgtg aggtctacaa a                                                 21

<210>  5
<211>  27
<212>  DNA
<213>  Artificial sequence

<220>
<223>  Primer

<400>  5
attcgctcta ctccaaccac ataatag                                          27


<210>  6
<211>  25
<212>  DNA
<213>  Artificial sequence

<220>
<223>  Primer

<400>  6
gctccataat cagaagatgc gctac                                            25


<210>  7
<211>  25
<212>  DNA
<213>  Artificial sequence

<220>
<223>  Primer

<400>  7
gcgtagctcc ataatcagaa gatgc                                            25


<210>  8
<211>  27
<212>  DNA
<213>  Artificial sequence

<220>
<223>  Primer

<400>  8
gtaggcgtag ctccataatc agaagat                                          27


<210>  9
<211>  24
<212>  DNA
<213>  Artificial sequence

<220>
<223>  Primer

<400>  9
gtagctccat aatcagaaga tgcg                                             24


<210>  10
<211>  24
<212>  DNA

<213>  Artificial sequence

<220>
<223>  Primer

<400>  10
tggtggtgat tacgtatctt agtc                                              24


<210>  11
<211>  24
<212>  DNA
<213>  Artificial sequence

<220>
<223>  Primer

<400>  11
cctagcctaa gttcagatgt aagg                                              24


<210>  12
<211>  24
<212>  DNA
<213>  Artificial sequence

<220>
<223>  Primer

<400>  12
tcatttctgc tagtctacct tagc                                              24


<210>  13
<211>  24
<212>  DNA
<213>  Artificial sequence

<220>
<223>  Primer

<400>  13
tcatttctgc tagtctacct tagc                                              24


<210>  14
<211>  22
<212>  DNA
<213>  Artificial sequence

<220>
<223>  Primer

<400>  14
tcgtacgggc tatccactct ag                                                22


<210>  15
<211>  24
<212>  DNA
<213>  Artificial sequence

<220>

<223>  Primer

<400>  15
ctatccactc tagcaaacga ctta                                              24


<210>  16
<211>  26
<212>  DNA
<213>  Artificial sequence

<220>
<223>  Primer

<400>  16
aggatgctat ttagtctact taagct                                            26


<210>  17
<211>  24
<212>  DNA
<213>  Artificial sequence

<220>
<223>  Primer

<400>  17
ccagatgccg aataagtgta gtta                                              24


<210>  18
<211>  24
<212>  DNA
<213>  Artificial sequence

<220>
<223>  Primer

<400>  18
gcccttaaga tcagatacaa caga                                              24


<210>  19
<211>  24
<212>  DNA
<213>  Artificial sequence

<220>
<223>  Primer

<400>  19
aagactagaa ctccgtgagt gata                                              24


<210>  20
<211>  24
<212>  DNA
<213>  Artificial sequence

<220>
<223>  Primer

<400>  20

atacacccgt acgtaatcac aatc 24

<210> 21
<211> 24
<212> DNA
<213> Artificial sequence

<220>
<223> Primer

<400> 21
gcaatttaat ccgcctactc atat 24

<210> 22
<211> 24
<212> DNA
<213> Artificial sequence

<220>
<223> Primer

<400> 22
atcaaccgta cgtttgtatt tcac 24

<210> 23
<211> 21
<212> DNA
<213> Artificial sequence

<220>
<223> Primer

<400> 23
cagcgttgcg aaacttatca t 21

<210> 24
<211> 24
<212> DNA
<213> Artificial sequence

<220>
<223> Primer

<400> 24
gacttttcgc acacatatga gtag 24

<210> 25
<211> 24
<212> DNA
<213> Artificial sequence

<220>
<223> Primer

<400> 25
cgtatttccg gtattctaat ccct 24

<210> 26
<211> 24
<212> DNA
<213> Artificial sequence

<220>
<223> Primer

<400> 26
gcacgtattt ccggtattct aatc 24


<210> 27
<211> 23
<212> DNA
<213> Artificial sequence

<220>
<223> Primer

<400> 27
atccgaggtt ctcacgaaat tag 23


<210> 28
<211> 22
<212> DNA
<213> Artificial sequence

<220>
<223> Primer

<400> 28
ctcgcatcaa caagtcgtaa ag 22


<210> 29
<211> 450
<212> DNA
<213> Artificial sequence

<220>
<223> Template

<400> 29
aaattcgctc tactccaacc acataatagt cctcacccaa acacactcgc cagttccgat 60

actaaggctc tgccggcgct ctctttgaga tatcggttcg aaatcgacca cagtataaac 120

ggatatagga acactgttag ttcgcatcca agcagtaggg gccagacctg tctcgctttg 180

tacgatactc ggttagtaga ccaatgccaa ctaaggggat gtgttcacgg catttcggag 240

ttcaaagggt tggtgagaag agctctcgaa gttgtttggg tgactcgatc ctaggaacct 300

acggcttatc gatacaaacc cacagctttc cgtccatgtg aagtatggga tgcgcaagac 360

ctgagcagcc gcgtgaggtc tacaaagttt aaaccgccgt tgtgtacggt agcgcatctt 420

ctgattatgg agctacgcct atcgctcgct 450


<210> 30

<211>  255
<212>  DNA
<213>  Artificial sequence

<220>
<223>  Template

<400>  30
agccaactca gtcaatctca aaccgttttc cggtgttggt ggtgattacg tatcttagtc      60

ccgctggccg ggtaatgata ccacgaatca attctcgaac tatgctgctt ccccgatccc     120

gccccgcctt aagactttat gctcacgcgt tacggctcga aagatagtcc aggcgagacg     180

tgtgtagagt tatgcgaaac gatctcagac gcgtttagcg gttaaaatgt cacacaaggg     240

cattacccga ccggg                                                      255


<210>  31
<211>  255
<212>  DNA
<213>  Artificial sequence

<220>
<223>  Template

<400>  31
cgaacggggt ccggtatctg accgtcacat gtgccatcct cttggtcgta cgggctatcc      60

actctagcaa acgacttact ttgacggtaa tggcaggccc ctaactacac ttattcggca     120

tctgggtgtt tacgatgacg ctcctcccgc tatggtcaag cgacaggagt ggagatcatc     180

gtacacgtcc caatgcatat attcacccgg ccacgtatcc gtgcaggttg ccgtgggtct     240

gggagtgaac gtgcg                                                      255


<210>  32
<211>  255
<212>  DNA
<213>  Artificial sequence

<220>
<223>  Template

<400>  32
tcccgccctt aagatcagat acaacagaaa gcaagcaccc cgcctaggaa cagccccgtt      60

gccgtcaatt actcttaatt ccaacggatt gttgctctcc ggagagaagg aaagtaagac     120

tgtcgccggg ttacgtgtgt ttgtatccaa atcgccgaga gactcgatga ccattcggga     180

gttgtaccca agaagccgcg ggtaaggcgt gcgggactca actcccaaat agtctcagtc     240

cctcgaggta tatag                                                      255


<210>  33
<211>  255
<212>  DNA
<213>  Artificial sequence

<220>
<223>  Template

<400>  33
gatcaaccgt acgtttgtat ttcacactta cacttgcaat ttaatccgcc tactcatatg      60

tgtgcgaaaa gtcggaggct agaactgtag cccgaatcgg ccgcaaggac ccactaaacg     120

cgtagttgat atttcaaccg tagtagttgg gttcccactt aggccgagcc gagaggtcct     180

ggatagtggt cagtagttaa gaggtgtctc ggtaagtata gagcaagtca cggccgaata     240

catgccaact ccccc                                                      255


<210>  34
<211>  255
<212>  DNA
<213>  Artificial sequence

<220>
<223>  Template

<400>  34
gggcacgtat ttccggtatt ctaatccctg catgagaggg cctccacatc aacacgcgtc      60

cagctttacg acttgttgat gcgagtgacc gtcgctatac tccgcttgcc cgttctgagg     120

ggaactcacg tatcggccct tgataatgcg ttcgtcacag ggttcctaaa gtcgggcaca     180

gtgagctgaa aggtgcatcc gtgccaaatg gaacctgtgg tggccacact cgggttaaac     240

tcagcgctag tgaaa                                                      255

## Description

Molecular biology techniques are widely used in genotyping applications and other areas such as biological research, forensic and diagnostic applications, including human identification and paternity testing and for diagnosis of infectious diseases or chimera analysis after allogeneic bone marrow transplantation as well the detection of genetic diseases and cancer. The most commonly used technique is the polymerase chain reaction (PCR) that allows the researchers to amplify the desired DNA requiring only tiny amounts of sample.

Forensic workflow schemes entail the amplification of so called short tandem repeat (STR) markers. These markers are genetic elements of variable lengths that are characterized by short repetitive sequence motifs and are used in combination with other STR loci in order to obtain a genetic fingerprint of an individual. This information can be further used to accurately identify or eliminate a suspect by comparing this fingerprint with evidence from the crime scene.

After amplification, the resulting PCR products are labelled using fluorescent dyes and the technique of capillary electrophoresis (CE) is employed to separate said amplification products according to their molecular size. The fluorescent signals are represented as peaks in the electropherogram.

However, such analyses are hampered by several practical issues that prevent accurate genotyping. At the level of the template, possible degradation of the sample complicates efficient amplification of the target sequence. Further, naturally occurring compounds such as hematin or humic acid are potent inhibitors of PCR reactions. Since forensic samples are usually only available in very small quantities, the extent of possible trouble shooting in order to obtain a conclusive result is very limited.

Current means to provide an internal amplification control are hampered by non-specific binding that complicates subsequent analysis and do not allow for the distinction of the presence of inhibitors and template degradation.

Therefore, there is a need for a system that allows the researcher to differentially identify said problems in amplification and detection experiments.

### Brief description of the invention

The invention addresses the technical problems described above by means of an internal amplification control consisting of a set of primers and a template.

The invention further relates to a method for evaluating the amplification efficiency and/or the presence of inhibitors and/or degradation in an amplification reaction comprising a reaction mixture comprising
i. one or more internal nucleic acid templates with a length of between 50 and 2000 nucleotides,
ii. at least 3 primers or 2 pairs of primers, which allow the generation of a first internal amplification product of between 20 and 800 nucleotides and at least one further internal amplification product of between 50 and 2000 nucleotides, wherein the at least 3 primers or 2 pairs of primers are specific for the one or more internal nucleic acid templates and the first and second internal amplification product are amplification products of the one or more internal nucleic acid templates, wherein the first internal amplification product is smaller than the second internal amplification product,
iii. a sample nucleic acid to be analysed and primers specific for said nucleic acid and reagents for said amplification,
wherein an amplification reaction is performed with said reaction mixture and the ratio of the smaller internal control amplification product to the larger internal control amplification product is analysed.

The invention further relates to a kit for evaluating the amplification efficiency and/or the presence of inhibitors and/or degradation in an amplification reaction, comprising
i. a nucleic acid to serve as an internal nucleic acid template and
ii. at least 3 primers that are specific for said internal nucleic acid template and wherein said primers generate a smaller and larger amplification product of the internal nucleic acid template and
iii. reagents for amplification and detection of nucleic acids.

### Detailed description of the invention

A "primer" herein refers to an oligonucleotide comprising a sequence that is complementary to a nucleic acid to be amplified or transcribed ("template"). During replication a polymerase attaches nucleotides to the 3'-OH end of the primer complementary to the respective nucleotides of the template.

The invention further relates to a method for evaluating the amplification efficiency and/or the presence of inhibitors and/or degradation in an amplification reaction comprising a reaction mixture comprising
i. one or more internal nucleic acid templates with a length of between 50 and 2000 nucleotides,
ii. at least 3 primers or 2 pairs of primers, which allow the generation of a first internal amplification product of between 20 and 800 nucleotides and at least one further internal amplification product of between 50 and 2000 nucleotides, wherein the at least 3 primers or 2 pairs of primers are specific for the one or more internal nucleic acid templates and the first and second internal amplification product are amplification products of the one or more internal nucleic acid templates, wherein the first internal amplification product is smaller than the second internal amplification product,
iii. a sample nucleic acid to be analysed and primers specific for said nucleic acid and reagents for said amplification,
wherein an amplification reaction is performed with said reaction mixture and the ratio of the smaller internal control amplification product to the larger internal control amplification product is analysed.

In particular the method relates to the use of an internal control nucleic acid and its amplification as an internal control.

In one embodiment the internal nucleic acid template or the one or more internal nucleic acid templates are artificial nucleic acid templates. In an alternative embodiment the internal nucleic acid template or the one or more internal nucleic acid templates consist of a sequence of an organism, which is different than the origin or suspected origin of the sample nucleic acid to be analyzed. In a preferred embodiment the internal nucleic acid template or the one or more internal nucleic acid templates consist of a sequence with a very low identity to sequences in the sample. In a preferred embodiment the internal nucleic acid template or templates have been generated using a random algorithm. In a more preferred embodiment the internal nucleic acid template or templates comprise any of the non-human artificial sequences SEQ ID NOs 29 to 34. In a most preferred embodiment the internal nucleic acid template or templates consist of a sequence selected from the group comprising SEQ ID NOs 29, 30, 31, 32, 33 and 34.

In a preferred embodiment the internal nucleic acid template or templates have a length between 70 and 2000 nucleotides. In a preferred embodiment the internal nucleic acid template or templates have a length between 150 and 1500 nucleotides. In a more preferred embodiment the internal nucleic acid template or templates have a length between 150 and 1000 nucleotides. In an even more preferred embodiment the internal nucleic acid template or templates have a length between 200 and 500 nucleotides.

More preferably the internal nucleic acid template or templates have a length between 50 and 2000 nucleotides. In a preferred embodiment the internal nucleic acid template or templates have a length between 50 and 1500 nucleotides. In a more preferred embodiment the internal nucleic acid template or templates have a length between 50 and 1000 nucleotides. In an even more preferred embodiment the internal nucleic acid template or templates have a length between 50 and 500 nucleotides.

The method relates to at least two internal amplification products, wherein one amplification product has a shorter length than the other. These amplification products are referred to herein as first and second amplification product. Preferably the first amplification product has a length of at least 20 nucleotides and the second amplification product has a length of at least 70 nucleotides. The difference in length of the first and second amplification product has to be detectable.

The invention further relates to primers or sets of primers to amplify the internal nucleic acid template or templates. If the internal nucleic acid template or templates comprise a sequence selected from SEQ ID NOs 29 to 34 it is preferred that the primers are selected from the group comprising SEQ ID NOs 1 to 28.

In one embodiment the invention relates to a method for evaluating the amplification efficiency and/or the presence of inhibitors and/or degradation in an amplification reaction, wherein the analysis of the ratio of said first smaller internal control amplification product to said second larger internal control amplification product is performed by a method selected from the group comprising agarose gel electrophoresis, capillary electrophoresis, qPCR, digital PCR and next generation sequencing. The use of capillary electrophoresis is preferred, wherein the resulting amplification products are separated according to their molecular size and fluorescent dye label. The fluorescent dyes are frequently used as molecular labels attached to probes or other biomolecules.

The amplification may be a multiplex PCR. Multiplex PCR is for example used for the simultaneous detection of multiple marker genes and/or their polymorphisms, e.g. short tandem repeats (STRs) or deletion insertion polymorphisms (DIPS or Indels). Detection of the amplification products and their genotyping is usually carried out by multiple colour fluorescence detection after electrophoretic separation (e.g. capillary gel electrophoresis) in DNA sequencers. In real-time, quantitative multiplex PCR, the amplification of multiple target sequences can be monitored at the same time by simultaneous detection of fluorescence of different fluorescent dyes.

The amplification reaction is preferably selected from the group comprising polymerase chain reaction (PCR), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR), QP amplification and (thermostable) helicase dependent amplification ((t)HAD). More preferably the amplification reaction is a PCR. Most preferably, the amplification reaction is a multiplex PCR, i.e. the amplification of more than one target nucleic acid sequence in a single tube, e.g. 2, 3, 4, 5, 6, 7, 8, 9, 10, 11, 12, 13, 14, 15,16, 17,18, 19, 20, 21, 22, 23, 24, 25, 26, 27, 28, 29, 30, 31, 32, 33, 34, 35, 36, 37, 38, 39 or 40 loci (target nucleic acid sequences) may be amplified simultaneously.

In another embodiment the invention relates to a method for evaluating the amplification efficiency and/or the presence of inhibitors and/or degradation in an amplification reaction, wherein the analysis of the ratio of said first smaller internal control amplification product to said second larger internal control amplification product is performed by a method called "Rapid DNA Analysis". Herein DNA from buccal and blood samples is processed by a fully automated DNA analysis system in about 90 minutes. Commercially available systems are for example RapidHIT™ (IntegenX), DNAscan (GE Healthcare Life Sciences, NetBio® Inc.). Rapid DNA Analysis Systems automatically extract DNA, perform the amplification of STR marker regions, separate the DNA fragments via capillary electrophoresis and produce standardized DNA profiles.

In a preferred embodiment the invention relates to a method for evaluating the amplification efficiency and/or the presence of inhibitors and/or degradation in an amplification reaction, wherein in addition several STR markers are amplified.

In a more preferred embodiment the invention relates to a method for evaluating the amplification efficiency and/or the presence of inhibitors and/or degradation in an amplification reaction, wherein the amplification reaction is performed in the context of genotyping. Such genotyping typically involves the amplification of a set of STR markers using multiplex PCR.

Analysis of the target amplification products and the internal control fragments allows for the differential identification of the presence of inhibitors or degradation of the sample.

The invention relates to a method for evaluating the amplification efficiency and/or the presence of inhibitors and/or degradation in an amplification reaction, wherein an equal ratio of the small and the larger internal control amplification products suggests a non-inhibited sample. Herein, an equal ratio of the small and the larger internal control amplification products together with a ratio in favour of small target products of the sample suggests a degraded sample. A shift of a ratio in favour of the small internal amplification product suggests the presence of one or several inhibitors in the sample. In the case that no target product is detected in the analysis of the amplification products, the presence of both the large and the small internal control amplification fragments suggests that the sample did not comprise amplifiable nucleic acids.

In a preferred embodiment the invention relates to a nucleic acid, wherein the nucleic acid comprising a sequence elected from the group consisting of SEQ ID NO: 1 to 28 is used as a primer or a probe. These sequences are used in amplification reactions to generate said two internal amplification products.

The invention relates to the use of a nucleic acid according to SEQ ID NO 29 to 34 as an internal nucleic acid template in an amplification reaction. Said template is preferably used in a multiplex PCR reaction, wherein a set of STR markers are analysed. Said two internal control products of different sizes are amplified using that internal template.

The invention relates to a kit for evaluating the amplification efficiency and/or the presence of degradation and/or degradation in an amplification reaction, comprising
i. one or more nucleic acid to serve as an internal nucleic acid template and
ii. at least 3 primers or at least two pairs of primers that are specific for said one or more internal nucleic acid templates and wherein said primers generate a smaller and larger amplification product of the one or more internal nucleic acid templates and
iii. reagents for amplification and detection of nucleic acids.

### Examples

Previous STR analysis protocols entailed the use of primers and plasmids that originate from other organisms than the one of the sample to be analysed. In other cases, the same primers were used in both the internal control reaction and in the multiplex amplification of a target genomic region. However, this strategy of said parallel usage of primers is risky. In the case of forensic samples where the presence of PCR inhibitors and template quantity is critical, a drop in performance is observed in the amplification of the genomic target region. Using components from other kits as an internal control bears the risk of cross contamination.

Further, current methods did not provide a conclusive insight into whether an amplification reaction is hampered by the presence of a degraded template or by PCR inhibitors in said template.

### Design and bioinformatics analysis of template and primer sequences

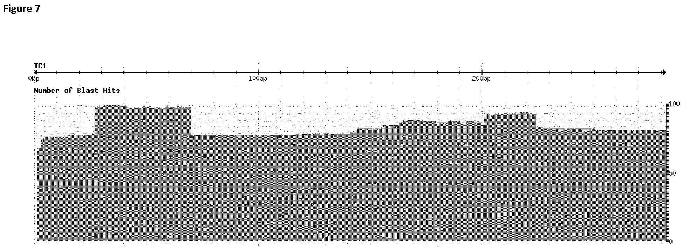

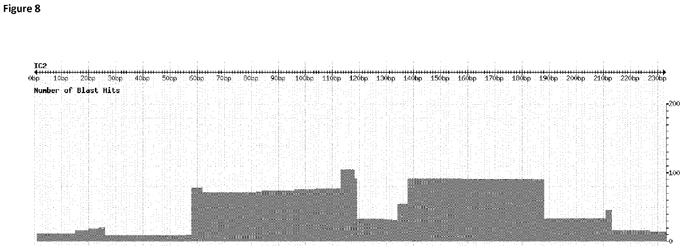

In order to assess sequence similarity of known internal control templates such as IC1 and IC2 to sequences available in public databases, BLAST searches were performed. As shown in Figure 7, in the case of IC1 at least 80 hits were identified. Some regions of the sequence show up to 100 hits. The BLAST search using IC2 also identified several hits across the sequence (Figure 8).

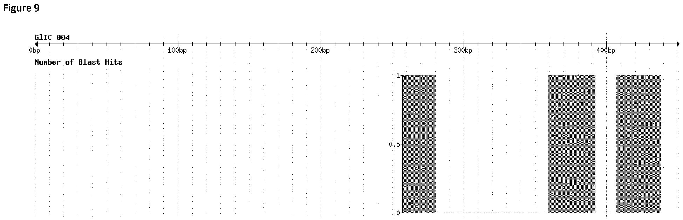

To address the aspect of sequence similarity and the possibility of non-specific binding synthetic DNA templates were designed using a random algorithm. A subsequent BLAST search yielded only up to 3 distinct hits spanning a short segment of 20 to 30 bp (Figure 9). The chance of non-specific binding in the context of a multiplex PCR amplification reaction is therefore very low. Primers (selected from SEQ ID NO 1 to 28) were designed based on these sequences to amplify two products of different sizes. The sequence of the primers and the first 30 nucleotides of the templates are shown in Table 1.

| **SEQ ID NO** | **Sequence annotation** | **Sequence** |
|---|---|---|
| 1 | GlIC_004_for1 | tactccaaccacataatagtcctc |
| 2 | GlIC_004_for2 | tctactccaaccacataatagtcc |
| 3 | GlIC_004_for3 | tcgctctactccaaccacataata |
| 4 | GlIC_004_for6 | cagccgcgtgaggtctacaaa |
| 5 | GlIC_004_for7 | attcgctctactccaaccacataatag |
| 6 | GlIC_004_rev1 | gctccataatcagaagatgcgctac |
| 7 | GlIC_004_rev2 | gcgtagctccataatcagaagatgc |
| 8 | GlIC_004_rev3 | gtaggcgtagctccataatcagaagat |
| 9 | GlIC_004_rev5 | gtagctccataatcagaagatgcg |
| 10 | GlIC_048_for1 | tggtggtgattacgtatcttagtc |
| 11 | GlIC_048_for2 | cctagcctaagttcagatgtaagg |
| 12 | GlIC_048_rev1 | tcatttctgctagtctaccttagc |
| 13 | GlIC_048_rev2 | tcatttctgctagtctaccttagc |
| 14 | GlIC_058_for1 | tcgtacgggctatccactctag |
| 15 | GlIC_058_for2 | ctatccactctagcaaacgactta |
| 16 | GlIC_058_rev1 | aggatgctatttagtctacttaagct |
| 17 | GlIC_058_rev2 | ccagatgccgaataagtgtagtta |
| 18 | GlIC_073_for1 | gcccttaagatcagatacaacaga |
| 19 | GlIC_073_for2 | aagactagaactccgtgagtgata |
| 20 | GlIC_073_rev1 | atacacccgtacgtaatcacaatc |
| 21 | GlIC_074_for1 | gcaatttaatccgcctactcatat |
| 22 | GlIC_074_for2 | atcaaccgtacgtttgtatttcac |
| 23 | GlIC_074_rev1 | cagcgttgcgaaacttatcat |
| 24 | GlIC_074_rev2 | Gacttttcgcacacatatgagtag |
| 25 | GlIC_082_for1 | cgtatttccggtattctaatccct |
| 26 | GlIC_082_for2 | gcacgtatttccggtattctaatc |
| 27 | GlIC_082_rev1 | atccgaggttctcacgaaattag |
| 28 | GlIC_082_rev2 | ctcgcatcaacaagtcgtaaag |
| 29 | GlIC_004 | aaattcgctctactccaaccacataatagt |
| 30 | GlIC_048 | agccaactcagtcaatctcaaaccgttttc |
| 31 | GlIC_058 | cgaacggggtccggtatctgaccgtcacat |
| 32 | GlIC_073 | tcccgcccttaagatcagatacaacagaaa |
| 33 | GlIC_074 | gatcaaccgtacgtttgtatttcacactta |
| 34 | GlIC_082 | gggcacgtatttccggtattctaatccctg |

**Table 1: Primer and template sequences.** The sequences of the primers and the templates are presented together with the corresponding SEQ ID NO and sequence annotation. Note that in the case of the templates (SEQ ID NO 29 to 34), only the first 30 nucleotides are presented. The complete sequences can be found in the sequence protocol.

Analysis of the presence of these two internal template amplification products allows the researchers to differentially identify the presence of PCR inhibitors or the level of degradation in the forensic sample. When inhibitors such as hematin and humic acid are present in a sample, amplification is less efficient and especially larger DNA fragments are amplified less than smaller ones. In the case of sample degradation, smaller fragments are more abundant and larger target fragments are also amplified less efficiently. However, said degradation of the target template does not hamper amplification of the internal control fragments from the internal control template.

### Validation of internal amplification control

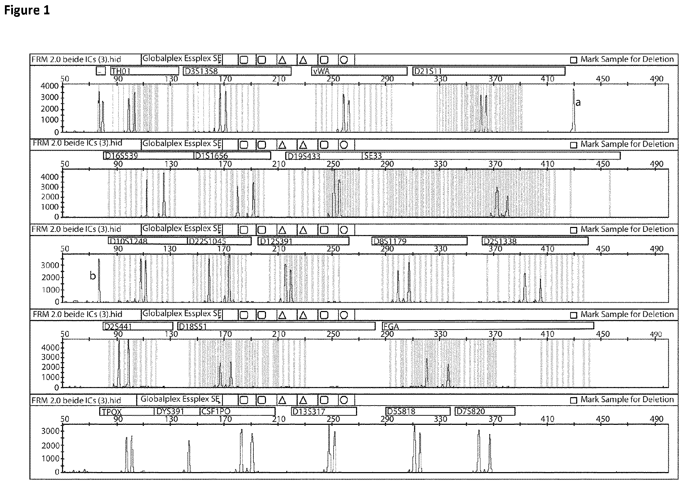

In order to validate said internal amplification template and primers in amplification experiments, STR analyses were performed. In the preliminary experiment, a set of 23 markers was amplified. As shown in Figure 1, all markers were amplified correctly and are represented in the electropherogram according to their molecular size and dye label. Further, both the large and the small internal control fragments were amplified correctly and can be identified in the electropherogram at the expected locations (Figure 1, a: large fragment, b: small fragment). This shows that the amplification reaction was successful and that the presence of the internal template and primers did not affect amplification of the target STR markers.

### Presence of inhibitors

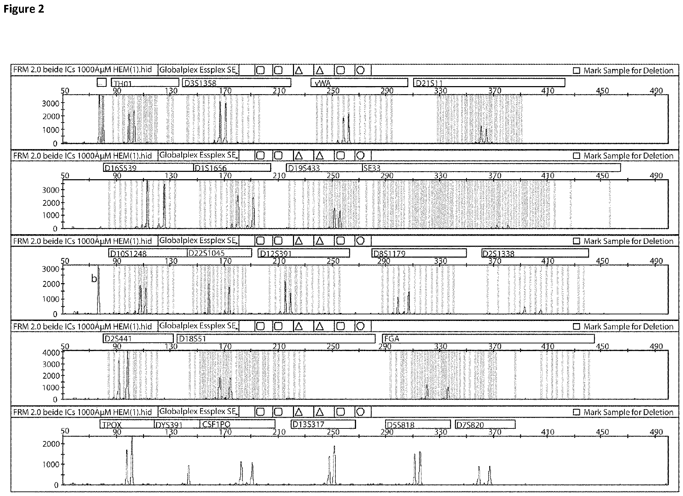

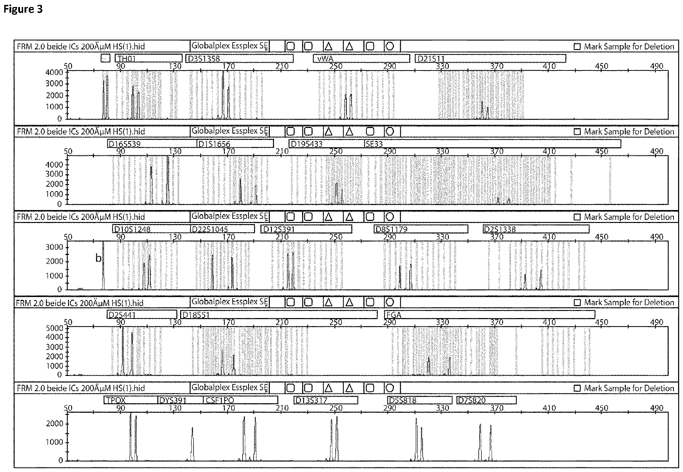

The subsequent experiments consisted of STR analyses in the presence of 1 mM hematin and 200 ng/µl humic acid. The presence of these compounds led to inefficient amplification of larger DNA fragments. The corresponding peaks in the electropherogram are either very small or not visible. Similarly, the larger internal control fragment was also not amplified efficiently. The small internal control fragment however was amplified and can be identified in the electropherograms (Figure 2 and 3, fragment b).

### Sample degradation

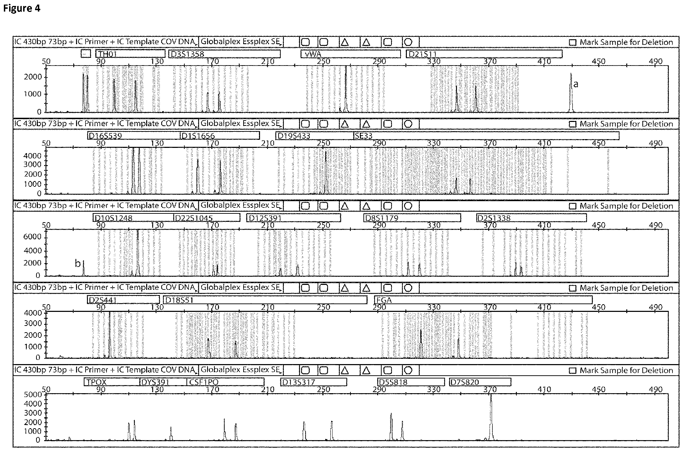

Further, the internal control primers and template were evaluated with regards to sample degradation. The preliminary experiment using intact genomic DNA showed that all STR markers were amplified and are represented in the electropherogram according to their molecular size and dye label. Both the large and the small internal control fragment were amplified (Figure 4, a and b).

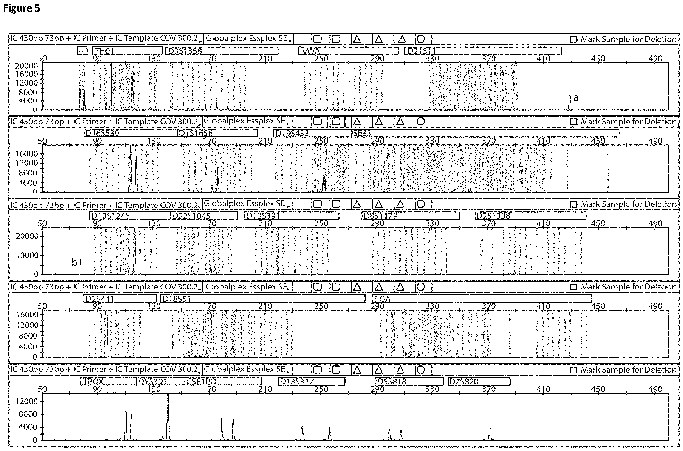

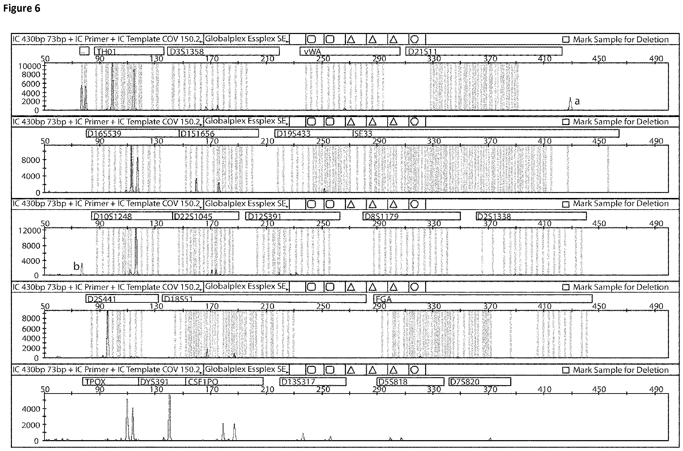

For the subsequent experiments said genomic DNA was sheared to fragments of 300 and 150 bp respectively using an ultra-sonic device (Covaris) and amplification reactions were performed. As shown in Figure 5 and 6, larger STR target fragments were not amplified efficiently and the corresponding peaks are either small or not present in the electropherograms. However, in both cases the large and the small internal control fragments were amplified irrespective of said degradation of the target template (Figure 5 and 6, a and b).

In summary, the examples show that a system comprising an internal artificial template together with three primers that amplify two fragments of distinct sizes from the internal control is a means to distinguish the presence of inhibitors from the DNA degradation level and to thereby evaluate amplification efficiency. Presence of inhibitors is characterized by inefficient amplification of both the larger target fragments and the larger internal control fragment whilst maintaining efficient amplification of the smaller internal control fragment. Degradation however does not affect efficient amplification of both internal control fragments.

### Figure captions

### Figure 1

**Electropherogram of STR analysis.** 23 STR markers were amplified and analysed using CE. The large and the small internal control fragments were amplified in the same reaction. Annotations are as follows: The respective markers are presented at the top of the electropherogram. a and b denote the large and the small internal control fragment respectively.

### Figure 2

**Electropherogram of STR analysis in the presence of hematin.** 23 STR markers were amplified in the presence of 1 mM hematin and analysed using CE. The large and the small internal control fragments were amplified in the same reaction. Annotations are as follows: The respective markers are presented at the top of the electropherogram. a and b denote the large and the small internal control fragment respectively. A peak corresponding to fragment a is absent.

### Figure 3

**Electropherogram of STR analysis in the presence of humic acid.** 23 STR markers were amplified in the presence of 200 ng/µl humic acid and analyzed using CE. The large and the small internal control fragments were amplified in the same reaction. Annotations are as follows: The respective markers are presented at the top of the electropherogram. a and b denote the large and the small internal control fragment respectively. A peak corresponding to fragment a is absent.

### Figure 4

**Electropherogram of STR analysis.** Genomic DNA was used in this experiment. Annotations are as follows: The respective markers are presented at the top of the electropherogram. a and b denote the large and the small internal control fragment respectively.

### Figure 5

### Electropherogram of STR analysis in the presence of DNA degradation (fragments of 300 bp).

Genomic DNA was used in this experiment and sheared to fragments of 300 bp using ultrasound. Annotations are as follows: The respective markers are presented at the top of the electropherogram. a and b denote the large and the small internal control fragment respectively.

### Figure 6

### Electropherogram of STR analysis in the presence of DNA degradation (fragments of 150 bp).

Genomic DNA was used in this experiment and sheared to fragments of 150 bp using ultrasound. Annotations are as follows: The respective markers are presented at the top of the electropherogram. a and b denote the large and the small internal control fragment respectively.

### Figure 7

**BLAST search using internal control IC1.** The sequence of IC1 is shown at the top. The number of BLAST hits is shown on the left. Grey areas represent the regions of the sequence per base that show hits in the BLAST search.

### Figure 8

**BLAST search using internal control IC2.** The sequence of IC2 is shown at the top. The number of BLAST hits is shown on the left. Grey areas represent the regions of the sequence per base that show hits in the BLAST search.

### Figure 9

**BLAST search using artificial template GIIC 004 (SEQ ID NO 29).** The sequence of GIIC 004 is shown at the top. The number of BLAST hits is shown on the left. Grey areas represent the regions of the sequence per base that show hits in the BLAST search.

## Claims

1. Method for evaluating the amplification efficiency and/or the presence of inhibitors and/or degradation in an amplification reaction comprising a reaction mixture comprising
i. one or more internal nucleic acid templates with a length of between 50 and 2000 nucleotides,
ii. at least 3 primers or at least 2 pairs of primers, which allow the generation of a first internal amplification product of between 20 and 800 nucleotides and at least one further internal amplification product of between 70 and 2000 nucleotides, wherein the 3 primers or 2 pairs of primers are specific for the one or more internal nucleic acid template and the first and second internal amplification product are amplification products of the one or more internal nucleic acid template, wherein the first internal amplification product is smaller than the second internal amplification product,
iii. a sample nucleic acid to be analysed and primers specific for said nucleic acid and reagents for said amplification,
wherein an amplification reaction is performed with said reaction mixture and the ratio of the smaller internal control amplification product to the larger internal control amplification product is analysed.

2. Method for evaluating the amplification efficiency and/or the presence of inhibitors and/or degradation in an amplification reaction according to claim 1, wherein the analysis of the ratio of said first smaller internal control amplification product to said second larger internal control amplification product is performed by a method selected from the group comprising agarose gel electrophoresis, capillary electrophoresis, qPCR, digital PCR and next generation sequencing.

3. Method for evaluating the amplification efficiency and/or the presence of inhibitors and/or degradation in an amplification reaction according to claim 1 or 2, wherein in addition several STR markers are amplified.

4. Method for evaluating the amplification efficiency and/or the presence of inhibitors and/or degradation in an amplification reaction according to claims 1 to 3, wherein the amplification reaction is performed in the context of genotyping.

5. Method for evaluating the amplification efficiency and/or the presence of inhibitors and/or degradation in an amplification reaction according to claims 1 or 2, wherein an equal ratio of the small and the larger products suggests degradation of the sample.

6. Method for evaluating the amplification efficiency and/or the presence of inhibitors and/or degradation in an amplification reaction according to claims 1-2, wherein a shift of said ratio in favour of the small product suggests the presence of one or several inhibitors.

7. A nucleic acid comprising a sequence selected from the group of SEQ ID NO 1 to 34.

8. A nucleic acid according to claim 7, wherein the nucleic acid comprises a sequence selected from the group of SEQ ID NO 29 to 34.

9. A nucleic acid according to claim 7, wherein the nucleic acid comprising a sequence selected from the group of SEQ ID NO 1 to 28 is used as a primer or a probe.

10. Use of a nucleic acid according to SEQ ID NO 29 to 34 as an internal nucleic acid template in an amplification reaction.

11. Kit for evaluating the amplification efficiency and/or the presence of inhibitors and/or degradation in an amplification reaction, comprising
i. one or more nucleic acids to serve as an internal nucleic acid template and
ii. at least 3 primers or at least two pairs of primers that are specific for said internal nucleic acid templates and wherein said primers generate a smaller and larger amplification product of the internal nucleic acid template and
iii. reagents for amplification and detection of nucleic acids.
